**Europäisches Patentamt**

**European Patent Office**

**Office européen des brevets**

(19)

(11) Veröffentlichungsnummer: **0 009 667**
**A1**

(12) # EUROPÄISCHE PATENTANMELDUNG

(21) Anmeldenummer: **79103369.9**

(51) Int. Cl.³: **A 61 M 15/00**

(22) Anmeldetag: **10.09.79**

(30) Priorität: **11.09.78 US 941434**

(43) Veröffentlichungstag der Anmeldung:
**16.04.80 Patentblatt 80/8**

(84) Benannte Vertragsstaaten:
**AT BE CH DE FR GB IT LU NL SE**

(71) Anmelder: **Newhouse, Michael T., Dr.**
**436 Queen Street South**
**Hamilton, Ontario(CA)**

(72) Erfinder: **Newhouse, Michael T., Dr.**
**436 Queen Street South**
**Hamilton, Ontario(CA)**

(74) Vertreter: **Hoffmann, Rudolf**
**c/o C.H. BOEHRINGER SOHN Patentabteilung**
**D-6507 Ingelheim am Rhein(DE)**

(54) **Inhalationsgerät.**

(57) Gerät (20) für die Inhalation von Medikamenten, das in Verbindung mit üblichen Aerosolabgabegeräten (26) benutzt wird. Das Inhalationsgerät (20) umfaßt eine Kammer (22), deren Volumen wesentlich kleiner ist als das menschliche Atemvolumen und die auf einer Seite eine Halterung (28,56) für das Aerosolabgabegerät (26) aufweist und an einer entfernten Stelle ein Mundstück (24). Das Medikament wird bei Betätigung des Aerosolabgabegerätes (26) in die Kammer (22) gesprüht und kann durch ein Einwegventil (30) zwischen Mundstück (24) und Kammer (22) aus dieser herausgesaugt werden.

Das Inhalationsgerät (20) bewirkt, daß von den Medikamententeilchen des Aerosols im wesentlichen nur diejenigen in den Körper des Patienten gelangen, welche die therapeutische Wirkung verursachen.

EP 0 009 667 A1

./...

FIG.1.

Die Erfindung betrifft ein Gerät für die Inhalation von Medikamenten zur Linderung von Erkrankungen des Respirationstrakts. Das Gerät wird in Verbindung mit üblichen Aerosolabgabegeräten angewendet.

Seit einiger Zeit sind verschiedene Medikamente in Aerosolabgabegeräten erhältlich, diese Geräte werden mit einfachen Mundrohren versehen, bei denen die Abgabe des Aerosols aus dem Vorratsbehälter und die Inhalation gleichzeitig erfolgen müssen.

Die üblichen Mundrohre weisen erhebliche Nachteile auf. Erstens kommt es zu einer unerwünschten Ablagerung beträchtlicher Mengen des Medikaments im Hals des Patienten; dies hat häufig unerwünschte Nebenwirkungen zur Folge. .
Zweitens muß der Patient Einatmung und Freisetzung des Medikaments koordinieren, damit das Medikament zur richtigen Zeit in den Atemstrom gelangt. Es ist aus diesen Gründen schwierig sicherzustellen, daß eine geeignete Dosis des Medikaments an den gewünschten Wirkungsort gelangt. Andererseits kann, wegen der Nebenwirkungen, eine vorsorgliche Erhöhung der Dosis pro Hub des Abgabegerätes bedenklich sein.

Die erforderliche Koordination ist ein wichtiger Faktor besonders bei Kindern, weil ihnen häufig die nötige Handfertigkeit oder Fähigkeit zur Koordination noch fehlt. Dementsprechend können die üblichen Geräte nur von solchen Patienten erfolgreich benutzt werden, die über eine gute Handfertigkeit und Koordinationsfähigkeit verfügen. Dadurch ist die Behandlung der Patienten zu sehr dem Zufall überlassen.

Es wurde nun gefunden, daß ein Gerät bereitgestellt werden kann, das in Verbindung mit Dosieraerosolabgabegeräten verwendet werden kann und gegenüber den bekannten Geräten eine wesentliche Verbesserung darstellt.

Das Gerät gemäß der Erfindung umfaßt eine Kammer, deren Volumen wesentlich kleiner ist als das normale Atemvolumen, und die einen Einlaß und einen Auslaß hat. Die Kammer legt den Weg des Atemstroms bei der Einatmung zwischen Einlaß und Auslaß (diese eingeschlossen) fest. Das Gerät ist mit einer Haltevorrichtung versehen, die das Aerosolabgabegerät hinsichtlich seiner Lage zu der Kammer fixiert und die Abgabe des Aerosols in die Kammer ermöglicht, und an der Seite des Auslasses ist die Kammer mit einem Mundrohr verbunden, das außer der Inhalieröffnung eine Abzugsöffnung hat. Die Auslaßöffnung der Kammer ist mit einem Ventil versehen, das sich beim Einatmen durch das Gerät automatisch öffnet und sich automatisch schließt, wenn durch die Abzugsöffnung ausgeatmet wird. Dementsprechend kann, nachdem das Medikament in die Kammer gespritzt wurde, das Medikament durch das Ventil inhaliert werden, indem der Patient normal durch das Mundstück einatmet.

Die Erfindung wird nun durch die Zeichnungen in Verbindung mit der nachstehenden Beschreibung näher erläutert.

Fig. 1 ist ein perspektivischer Schnitt durch eine bevorzugte Ausführungsform des erfindungsgemäßen Geräts, in das das Aerosolabgabegerät teilweise eingeschoben ist;

Fig. 2 ist ein vereinfachter Längsschnitt durch das Gerät bei der Ausatmung;

Fig. 3 ist ein Längsschnitt entsprechend Fig. 2 bei der Einatmung;

Fig. 4 und 5 zeigen Längsschnitte durch andere Ausführungsformen der Erfindung.

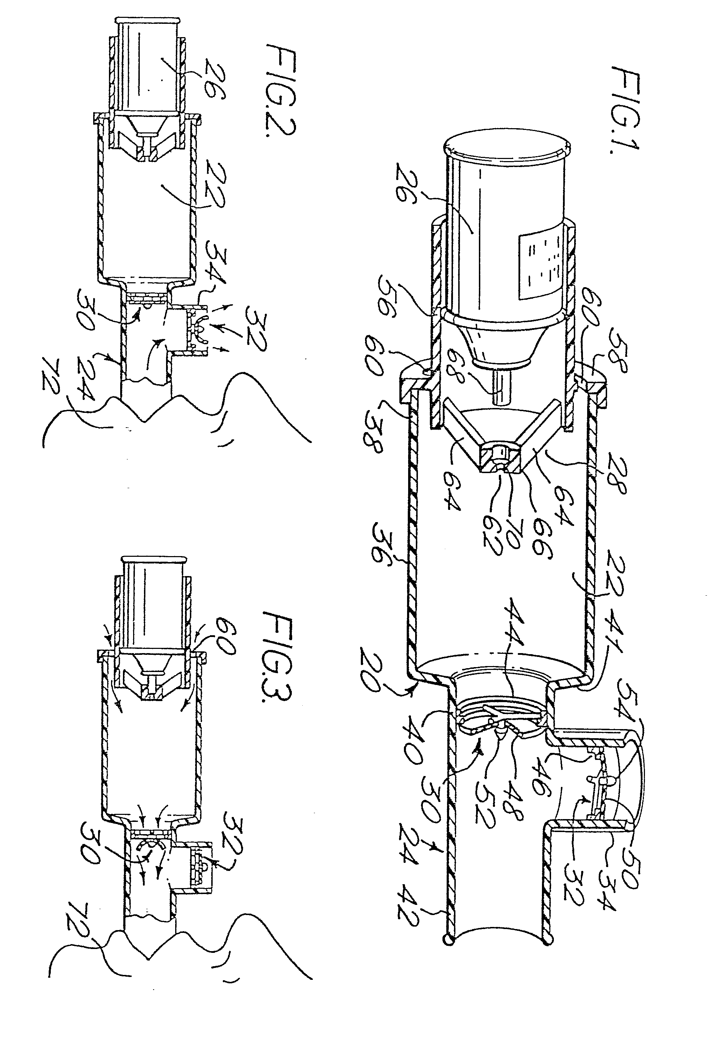

Fig. 1 veranschaulicht ein Gerät 20, welches die
Kammer 22, ein damit verbundenes Mundstück 24, durch
welches das aus dem Abgabegerät 26 freigesetzte
Medikament eingeatmet wird, und eine Halterung 28 umfaßt, die das Abgabegerät in seiner Position zur Kammer
22 fixiert. Ein erstes Ventil 30 erlaubt die Inhalation
durch die Kammer 22 zum Mundstück 24, während es die
Ausatmung durch das Mundstück in die Kammer 22 verhindert. Ein zweites Ventil 32 erlaubt die Ausatmung
durch das Gerät, verhindert jedoch die Einatmung durch
den Entlüftungsstutzen 34. Die Kammer 22 besteht aus
einer zylindrischen Wand 36 und umfaßt ein Endteil 38,
an dem die Halterung 28 für das Aerosolabgabegerät befestigt ist, wobei die Halterung 28 die Position des
Abgabegeräts bezüglich der Kammer 22 fixiert. Durch
die Endwandung 41 ist ein zylindrisches Teil 40 mit
der Wandung 36 der Kammer verbunden. Das zylindrische
Teil 40 bildet die Verlängerung des Mundstücks 24,
dessen äußeres Ende 42 eine für die Benutzung durch den
Patienten (entsprechend Fig. 2 und 3) geeignete Form
hat. Das Mundstück 24 umfaßt auch einen röhrenförmigen
Auslaßstutzen 34 mit dem Ventil 32. Die Ventile 30 und
32 haben ähnliche Form und bestehen jeweils aus einem
äußeren Rahmen 44, 46 mit Stegen und einem zentralen
Zapfen 52, 54, an denen die dünnen, elastischen Ventilscheiben 48,50 beweglich befestigt sind. Um die Herstellung des Geräts zu vereinfachen, werden zweckmäßig
die fertigen Ventile in das Gehäuse eingesetzt und
gegebenenfalls befestigt. Aus der Anordnung der Ventile
ergibt sich, daß der Luftstrom beim Einatmen durch die
Kammer in das Mundrohr gelangen muß, während er beim
Ausatmen den Weg durch den Auslaßstutzen 34 nimmt.

Die Halterung 28 ist so gestaltet, daß sie einen wesentlichen Teil des Aerosolabgabegeräts aufnimmt und durch Friktion in der gewünschten Position zur Kammer 22 festhält. Die Halterung 28 besteht im wesentlichen aus einem zylindrischen Hauptteil 56, der so dimensioniert ist, daß er das Abgabegerät locker aufnimmt. Der zylindrische Hauptteil 56 weist einen äußeren Rand 58 mit Einlaßöffnungen 60 auf sowie ein zentrales Befestigungselement 62, das von 3 Stegen 64 getragen wird, welche von der Innenseite des Hauptteils 56 ausgehen. Der Rand 58 ist so geformt, daß er den Teil 38 der Kammer 22 dicht umfaßt. Die Fixierung des Rands 58 an der Kammer 22 kann mechanisch erfolgen, so daß die Halterung 28 bei Bedarf auch von der Kammer 22 getrennt werden kann. Beide Teile können jedoch auch, z.B. durch Verkleben, unlösbar miteinander verbunden werden. Jedoch ist eine lösbare Steckverbindung zu bevorzugen.

Das Befestigungselement 62 in der Mitte der Halterung 28 weist eine größere Öffnung 66 auf, in die der mit einer achsialen Auslaßöffnung versehene Ventilstamm 68 des Abgabegeräts 26 hineingesteckt und durch Friktion festgehalten wird. Die Öffnung 66 endet in einer engeren Öffnung 70, durch welche das Aerosol in die Kammer 22 gesprüht werden kann.

Die Fig. 2 und 3 zeigen das Gerät 20 im Gebrauch. In Fig. 2 ist zu sehen, wie ein Patient 72 durch das Mundstück 24 und den damit verbundenen Auslaßstutzen ausatmet. Das Ventil 32 hat sich automatisch geöffnet, während der durch das Ausatmen erzeugte Druck das Ventil 30 in der geschlossenen Position hält. Mit dem vorliegenden Gerät ist es möglich, eine definierte Dosis aus dem Abgabegerät 26 in die Kammer 22 abzugeben. Dies geschieht in der Weise, daß das äußere Ende des Abgabegeräts 26 nach innen gedrückt wird, wodurch das in ihm befindliche Dosierventil konventioneller Art betätigt wird. Eine Medikament-Dosis gelangt dann als Aerosol in die Kammer 22 und steht dem Patienten für die Inhalation zur Verfügung. Das Gerät erlaubt es, daß der Patient normal durch das Gerät atmen und das Abgabegerät während des Ausatmens betätigen kann, wie in Fig. 2 dargestellt. Es ist jedoch auch möglich, die Medikament-Dosis in die Kammer 22 zu sprühen, kurz bevor der Patient das Gerät an den Mund setzt. So können Patienten mit verminderter Geschicklichkeit das Abgabegerät betätigen, indem sie das Gerät mit dem äußeren Ende des Abgabegeräts gegen einen geeigneten Gegenstand, z.B. gegen einen Tisch drücken. Dadurch wird eine Dosis in die Kammer 22 abgegeben und der Benutzer kann dann das Gerät an den Mund setzen, während er normal weiteratmet. Beim nächsten Einatmen bewegen sich die Ventile in die Position, die in Fig. 3 dargestellt ist; Ventil 32 ist geschlossen, Ventil 30 offen. Dementsprechend strömt die Atemluft beim Einatmen durch die Einlaßöffnungen 60 der Halterung 28, durch die Kammer und das Mundstück in den Respirationstrakt des Patienten und transportiert dabei das Arzneimittel in die Lunge. Wenn auch die Applikation des Arzneimittels durch normales Atmen durch das Gerät hervorgehoben wurde, so ist es selbstverständlich jedoch auch möglich, nach der Inhalation das Gerät abzusetzen und normal auszuatmen.

Das Gerät 20 ist einfach konstruiert und daher so preiswert, daß es, obgleich für mehrere Abgabegeräte benutzbar, auch als Einweggerät verwendet und jeweils mit dem leeren Abgabegerät weggeworfen werden kann.

Unerwarteterweise wurde gefunden, daß dadurch, daß die Medikament-Dosis erst in die Kammer gesprüht und dann aus dieser inhaliert wird, das Verhältnis von erwünschter Wirkung und unerwünschten Nebenwirkungen, gegenüber der direkten Inhalation aus üblichen Aerosolabgabegeräten, beträchtlich verbessert wird.

Bei der direkten Inhalation gelangen etwa 7,2 % der abgegebenen Dosis in die Lunge, während über 50 % im Hals abgelagert werden. Diese Zahlen gelten für einen normalen Patienten. Im Fall von Asthmapatienten wurde gefunden, daß der Anteil der Dosis, der die Lungen erreicht, auf etwa 6,2 % reduziert ist und im Hals über 50 % zurückgehalten werden. Im Unterschied dazu gelangen bei Benutzung des erfindungsgemäßen Geräts etwa 6,6 % der Dosis in die Lungen eines normalen Patienten, während etwa 3 % im Hals zurückgehalten werden. Bei Patienten mit obstruktiven Atemwegserkrankungen wird der Anteil der Dosis, der die Lungen erreicht, nur auf 6,2 % reduziert und im Hals werden ca. 3,5 % zurückgehalten. Es zeigt sich also, daß die bemerkenswerte Verminderung der Ablagerung im Hals nicht mit einer wesentlichen Verminderung des Dosisanteils erkauft wird, der die Lunge erreicht. Der Vorteil, den die Benutzung des erfindungsgemäßen Geräts mit sich bringt, ist also signifikant.

Die unerwarteten Vorteile dürften darauf zurückzuführen sein, daß die gröberen Teilchen, die das Abgabegerät freisetzt, an den Wänden und durch das Ventil zurückgehalten werden. Bei manchen Medikamenten werden flüssige Träger verwendet und daher kleine Tröpfchen versprüht. Ein Teil von diesen schlägt sich an den Wandungen der Kammer nieder, während andere trocknen, wobei sehr feine Partikel übrigbleiben, die von der Atemluft durch Hals und Luftröhre mitgerissen werden und die Lunge erreichen.

Die Abmessungen der Kammer und ihre Lage relativ zum Abgabegerät haben für ein gegebenes Abgabegerät auch einen Einfluß auf das Ergebnis. Ist die Kammer zu klein, wird ein unerwünscht hoher Anteil der Dosis zurückgehalten, während bei einer zu großen Kammer unerwünscht große Partikel in den Atemstrom gelangen können.

8

Als Beispiel für eine geeignete Ausführungsform ist das Gerät nach Fig. 1 zu bewerten. Mit einem Abgabegerät, das als Treibgas 20 % Freon 12 und 80 % Freon 114 verwendete, wurde gefunden, daß die lichte Länge der Kammer 22 zwischen etwa 8 und 10, der lichte Durchmesser zwischen etwa 3 und 4 cm betragen sollte.

Wie die obigen Darlegungen zeigen, ist das erfindungsgemäße Gerät einfach herzustellen, preisgünstig und selbst von solchen Personen einfach zu handhaben, die mit der Koordination von Atmung und Aerosol-Freisetzung Schwierigkeiten haben. Ferner hat sich gezeigt, daß die Größe der Partikel, die von der Atemluft mitgerissen werden, in dem günstigen Größenbereich von 1 bis 5 µm liegen.
Diese Teilchengröße ist optimal und hat zur Folge, daß die unerwünschte Ablagerung im Hals, verglichen mit Geräten nach dem Stand der Technik, entscheidend vermindert wird. Das erfindungsgemäße Gerät kann daher von einem sehr großen Teil der Patienten angewendet werden, einschließlich der Kinder und der Personen mit unzureichender Geschicklichkeit.

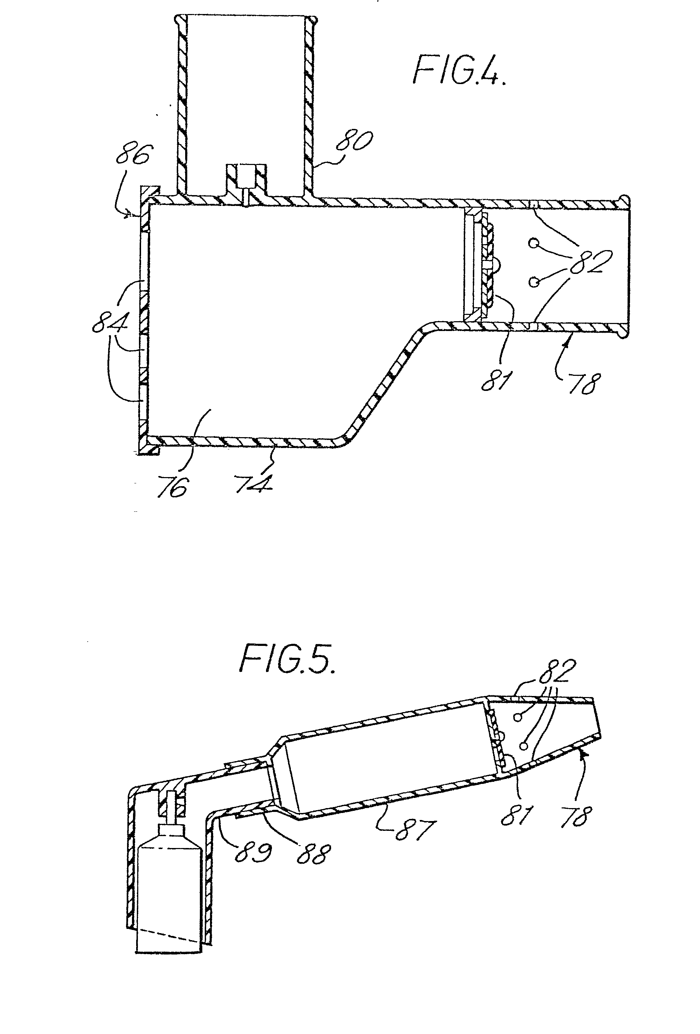

Es ist offensichtlich, daß das Gerät, wie es oben beschrieben wurde im Rahmen des Erfindungsgedankens in vielerlei Weise variiert werden kann. Zum Beispiel könnte es gegebenenfalls vorgezogen werden, das Abgabegerät vertikal statt horizontal anzuordnen und das Ventilsystem anders auszuführen. Eine solche Variante wird nun unter Bezugnahme auf Fig. 4 beschrieben.

Wie Fig. 4 zu entnehmen ist, ist ein Gerät 74 vorgesehen, das eine Kammer 76, ein Mundstück 78 und eine Halterung 80 aufweist. Diese Teile entsprechen funktionell denjenigen, die in Fig. 1 so bezeichnet sind. Auch ist ein Ventil 81, das dem Ventil 30 ähnelt,

9

vorgesehen, jedoch sind anstelle des Auslaßstutzens und des Ventils 32 der Fig. 1 eine Anzahl kleinerer Öffnungen 82 angebracht, die in der Wand des Mundstücks den Luftauslaß bilden. Während der Inhalation verläuft der Luftstrom überwiegend durch die Einlaßöffnungen 84 in einer Verschlußkappe 86 der Kammer 76 und dann durch das Ventil 81, weil der Widerstand auf diesem Weg wesentlich geringer ist als durch die Auslaßöffnungen 82. Ein gewisser Teil der Atemluft wird jedoch auch durch die Öffnungen 82 in das Mundstück gesaugt und trocknet, wie vorher erwähnt, das Medikament zusätzlich. Die Ausatmung erfolgt durch die Auslaßöffnungen 82. Diese Ausführungsform benutzt nur 1 Ventil und mag in manchen Fällen zu bevorzugen sein.

In einer anderen Ausführungsform ist das Gerät gemäß der Erfindung so gestaltet, daß es auf übliche Mundrohre für Aerosolabgabegeräte aufgesteckt werden kann. Ein Gerät dieser Art ist in Fig. 5 abgebildet, die einen Längsschnitt zeigt. Im Prinzip ähnelt dieses Gerät demjenigen nach Fig. 4. Jedoch weist das Gerät 87 anstelle der Halterung für das Abgabegerät einen Adapter 88 auf, mit dem es auf das bekannte Mundrohr 89 aufgesteckt und befestigt werden kann. Die anderen Teile entsprechen Fig. 4; es ist ein Mundstück 78 vorgesehen, in dem Auslaßöffnungen 82 für die Ausatmung und ein Ventil 81 für die Einatmung angebracht sind.

Um die Abmessungen des erfindungsgemäßen Geräts, beispielsweise der Ausführungsform nach Fig. 1 oder Fig. 5, zu reduzieren, kann es vorteilhaft auch teleskopartig gestaltet werden.

Patentansprüche

1. Gerät zur Verwendung in Verbindung mit Dosieraerosolabgabegeräten, gekennzeichnet durch eine Kammer mit
einem Einlaß und einem Auslaß und einem Volumen, das
wesentlich kleiner ist als das normale menschliche
Atemvolumen, wobei die Kammer mit einer Halterung,
welche das Abgabegerät für die Medikament-Abgabe
in einer bestimmten Position zur Kammer fixiert, sowie mit einem Mundstück verbunden ist, wobei das
Mundstück sich auf der Seite des Auslasses der
Kammer befindet und einen Luftauslaßstutzen aufweist,
daß ein erstes Ventil am Auslaß zwischen der Kammer
und dem Mundstück angebracht ist, welches sich beim
Einatmen durch das Gerät automatisch öffnet und beim
Ausatmen automatisch schließt, und daß ein zweites
Ventil mit dem Auslaßstutzen des Mundstücks in der
Weise verbunden ist, daß es sich beim Einatmen durch
das Gerät automatisch schließt und beim Ausatmen
automatisch öffnet, so daß die Applikation eines
Arzneimittels in Aerosolform beim normalen Atmen
durch das Mundstück möglich ist.

2. Gerät nach Anspruch 1, dadurch gekennzeichnet, daß das
Mundstück anstelle des Luftauslaßstutzens mit Ventil kleine Öffnungen aufweist, die den Luftdurchtritt in beiden Richtungen ermöglichen, die jedoch
eine solche Größe haben, daß beim Einatmen durch das
Mundstück der Hauptanteil der Atemluft durch die
Kammer und das Ventil an ihrem Auslaß in den Mund
des Patienten gelangt.

3. Gerät nach Anspruch 1 oder 2, dadurch gekennzeichnet,
daß der Halter für das Aerosolabgabegerät durch einen
Adapter ersetzt ist, mit dem sich das erfindungsgemäße Gerät auf einem üblichen Mundstück für Aerosolabgabegeräte durch Aufstecken fixieren läßt.

4. Gerät nach Anspruch 1 bis 3, dadurch gekennzeichnet, daß es teleskopisch gestaltet ist.

FIG.3

FIG.2

FIG.1

1/2

0009667

FIG.4.

FIG.5.

# EUROPÄISCHER RECHERCHENBERICHT

EP 79 103 369.9

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl.³) |
|---|---|---|---|
| | DE - A1 - 2 442 774 (K.P. UHLE)<br>* Seiten 1 bis 4; Fig., Positionen 2, 3, 4, 5, 10, 13 *<br>-- | 1 | A 61 M 15/00 |
| | GB - A - 975 754 (PFIZER LTD.)<br>* Ansprüche 1 bis 3; Seite 2, Zeilen 10 bis 17; Fig., Position 1 *<br>-- | 1 | |
| | US - A - 3 809 084 (L.F. HANSEN)<br>* Spalte 5, Zeilen 25 bis 50; Fig. 5, Position 46 *<br>-- | 1,3 | RECHERCHIERTE SACHGEBIETE (Int. Cl.³)<br><br>A 61 M 15/00 |
| | DE - A1 - 2 415 360 (AMERICAN CYANAMID CO.)<br>* Anspruch; Seiten 1 bis 5; Fig. 1, 3, Position 11 *<br>-- | 1 | |
| | US - A - 3 994 421 (L.F. HANSEN)<br>* Fig. 3 *<br>-- | 1 | |
| | GB - A - 1 017 032 (AEROSMOKE LTD.)<br>* Seite 2, Zeilen 100 und 101; Fig. 1, Positionen 6, 7 *<br>---- | 4 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 18-12-1979 | DROPMANN |

EPA form 1503.1 06.78